# EUROPEAN PATENT SPECIFICATION

(11) **EP 3 152 567 B1**
(45) Date of publication and mention of the grant of the patent: **05.12.2018**
(21) Application number: 15725050.7
(22) Date of filing: 29.05.2015
(51) Int. Cl.: G01N 33/28, G01N 22/00

(54) **CUT-OFF FREQUENCY ADJUSTMENT FOR MICROWAVE RESONATOR**
CUT-OFF FREQUENZ ANPASSUNG FÜR EINEN MIKROWELLENRESONATOR
RÉGLAGE DE LA FRÉQUENCE DE COUPURE POUR UN RÉSONATEUR À MICRO-ONDES

(30) Priority: 03.06.2014 NO 20140689
(43) Date of publication of application: 12.04.2017
(73) Proprietor: Roxar Flow Measurement AS, 4065 Stavanger (NO)
(72) Inventor: NYFORS, Ebbe Gustaf, N-4309 Sandnes (NO)
(74) Representative: Protector IP Consultants AS
(86) International application number: PCT/EP2015/061953
(87) International publication number: WO 2015/185450

(56) References cited:
- WO-A1-2008/085065
- US-A- 4 651 085
- US-B2- 6 826 964
- US-B2- 6 915 707

## Description

The present invention relates in general to measurements of fluid flow in a pipeline.

More specifically the present invention relates to measurement of water volume fraction (WVF), liquid film thickness, liquid film flow speed and/or salinity in a fluid flow, particularly in a wet gas or multiphase flow in a pipeline for transporting hydrocarbon-containing fluids, which in many cases additionally contains water, salt or other substances occurring in the exploration of oil and gas reservoirs.

A number of different commercial flow meters are available on the market for the measurement of the composition and properties of a mixture of oil, water and gas. Some meters are based on the use of radioactive radiation, some are capacitive, and some are based on the use of microwaves.

Microwave sensors are attractive because they are not limited by the health risks associated with radioactive radiation based meters and their fairly low accuracy or the undesirable influence of contamination on the capacitive sensors. An example is the wet gas flow meter described in NO 315584/US 6915707, where a structure in the pipe acts both as a device for creating a differential pressure and as a microwave cavity resonator sensor. This cavity resonator measures the permittivity of the whole volume of the flow, where the liquid (water and oil) mainly forms droplets in the gas. This is called mist flow. Another example of this type of sensors, and which relates to the present invention, is discussed in NO 328801/US8570050. The sensor is in principle a cavity resonator consisting of a piece of waveguide, which is shorted in on one end and open in the other. The waveguide is filled with a dielectric material and mounted in the wall of the spool piece to be as flush with the pipe wall as possible considering that the front is flat. Any liquid in the wet gas flow travelling as a film on the wall will also flow over the front of the sensor. This liquid will be in contact with the fringing capacitive electric field of the resonance and thereby affect both the resonant frequency (fr) and the Q-factor. The two main variables are the amount of water and the conductivity of the water. The conductivity depends on the salinity (salt content) of the water and the temperature. By measuring both fr and Q (in addition to the temperature) the salinity can be derived.

The advantages with this method of measuring salinity of a liquid forming a surface film, compared to methods based on measuring the complex permittivity of a volume of a flow, is that the changes in fr and Q are monotonous as a function of salinity. For the case of measuring in a volume, the water will typically be distributed as droplets (e.g. mist flow), and it can be shown that the imaginary part of the complex permittivity, caused by the conductivity in spherical droplets, first increase as the conductivity increases, but then start to decrease again.

The resonance mode in the cavity resonator sensor is based on the waveguide mode TM01 of a cylindrical waveguide. The field pattern is circularly symmetrical. Therefore, the open end does not radiate into the space in front of it. It has a very high Q-factor also when measured e.g. on the lab desk. However, any small disturbance of the symmetry, as e.g. some small water droplets on the front surface of the sensor, will cause radiation to occur. This results in a drop in the Q-factor, but this drop in the Q-factor is not caused by salt or conductive loss, rather due to asymmetry introduced in the sensor surface. When, however, the sensor is mounted in the wall of a metallic pipe, said pipe used for the transportation of a mixture of hydrocarbons and water, e.g. the spool piece of a so-called wet gas or multiphase flow meter, the drop in Q-factor due to sensor asymmetry introduced by droplets can be prevented by designing the sensor such that the resonant frequency of the senor is lower than the cut-off frequency for microwaves of the pipe under all flow conditions. This happens because microwaves at the resonant frequency then cannot propagate in the pipe and therefore cannot escape from the sensor.

The resonant frequency is mainly determined by the diameter of the cavity resonator sensor, if the resonator is constructed in a cylindrical profile with a circular cross-section facing the fluid flow. The area of the resonator facing the fluid flow may be termed as the aperture of the resonator. If the resonator is constructed in any other geometrical profile, such as cubical, cuboidal or prismatic, the largest dimension of the aperture of the resonator will typically define the resonant frequency. The resonant frequency of the cavity resonator further depends on the permittivity of the dielectric filling material inside the resonator. The height, or the dimension perpendicular or near perpendicular to the aperture, also has a minor effect on the resonant frequency, and it further influences the sensitivity of the sensor. It turns out that for any low value of the permittivity of the filling material, the sensor has to have an impractically large diameter to have a resonant frequency, which is lower than the cut-off frequency of the pipe. For this reason, and because of compatibility reasons, a ceramic material, for example using zirconia which has a permittivity of 28, may be preferable.

Since the dimensions of the cavity resonator sensor have to be chosen based on the cut-off frequency of the pipe, which depends on the inner diameter of the pipe, the microwave resonator sensor needs to be scaled with the pipe size. Doing this in practice would lead to an impractically large number of versions of sensors that need to be made for many different pipe sizes. Moreover, because resonators scaled according to different pipe diameters will operate at different frequencies, and since the permittivity of water is frequency dependent, there will need to be equally many different mathematical models for the calculation of the salinity, and all different sensors will typically have to be calibrated separately in a test flow loop.

The conclusion, to this end, is that the resonant frequency of the cavity resonator sensor must be lower than the cut-off frequency of the pipe. Besides this, another limitation is how large a resonator or sensor can be allowed to be from a mechanical/practical point of view. With zirconia as a material, there is some space between these limits. It is e.g. possible to cover pipe sizes from 2" to 8" with sensors with three different diameters. Considering that it may also be desirable to have sensors with different sensitivity for different applications, as e.g wet gas with low water content (needs high sensitivity), or multiphase flow with higher water content. Hence, the total number of versions of sensors needed to cover the entire product range with different pipe sizes and different sensitivities may become very large. Thus, it is an objective of the present invention to provide a more flexible solution and also reducing the number of different sensors.
Pipe sizes even larger than 8" may occur e.g. when the flow from several wells are combined and transported in a single pipe. Then none of the above-mentioned three sizes of sensors can be used. A sensor designed to fit large pipe sizes would not only be impractically large, but also expensive to manufacture. Thus, it is another objective of the present invention to provide a solution that can be used for large pipe sizes.

A related sensor assembly for measuring dielectric properties in a hydrocarbon fluid flow in a pipe is disclosed in US 6 826 964.

The invention is thus aimed at a salinity sensor constituting a microwave cavity resonator sensor to be mounted in the wall of a pipe or similar, as discussed in the abovementioned US patent US8570050. The resonant frequency of the sensor is below the cutoff frequency of the pipe so as to avoid loss by radiation e.g. caused by water droplets on the sensor that may be interpreted as caused by salinity.

As the pipe cutoff frequency decreases with an increasing pipe diameter, a larger pipe will have a lower cutoff frequency. The resonant frequency of the cavity resonator sensor depends on the sensor diameter and thus a larger diameter will give a low resonant frequency. Thus, a larger pipe diameter will require a larger sensor diameter. On large pipes this, however, this may not be practical. Thus, the main objective of the present invention is to provide microwave sensors with an increased range of use. This and other limitations of the prior-art will be shown solved by the following description and accompanying claims.

For the sake of simplicity and without limitation or loss of generality we will use the terms such as microwave cavity resonator, microwave resonator, resonator, cavity resonator sensor, or sensor interchangeably. We will further use the term resonator diameter, without limitation or loss of generality of the geometrical shape of the resonator or sensor, to imply the largest dimension along the aperture of the sensor or resonator. As discussed previously, the resonant frequency of said sensor or resonator is dependent on the largest dimension along the aperture of said sensor. Diameter, thereby, implies the largest dimension of a circular surface, which for a rectangular or square surface will be the length of the diagonal. For simplicity we will, therefore, use the terms such as diameter of the sensor and largest dimension of the sensor interchangeably to imply the largest dimension along the aperture of the sensor or resonator.

The invention solves this objective by providing an insert in front of the sensor delimiting part of the pipe so that the sensor sees a pipe section with a higher cut-off frequency than the main pipe. This way it is possible to use a small sensor in a large pipe.

Although the present discussion of the invention mainly refers to salinity measurements this is based on a specific embodiment and it is clear to a person skilled in the art that the invention may be used for other measurements as well. As mentioned above the sensor may be used, for example, for water volume fraction (WVF), liquid film thickness, liquid film flow speed and/or salinity in a fluid flow, or other measurements related to the permittivity of the flow.

The invention will be described below with reference to the accompanying drawings, illustrating the invention by way of examples.
- Figure 1-6: illustrate the cross section of a pipe with some examples of different shapes of plates forming a dedicated channel acting as a local waveguide over the open end of the cavity resonator sensor.
- Figure 7: shows a side-view of the system illustrating the plate, according to the present invention, forming the dedicated channel along the pipe length.

As is illustrated in the drawings, the present invention provides a local cut-off frequency in the volume 4 in front of the cavity resonator 2, said volume 4 defined by the aperture of the cavity resonator 2, the metallic wall 3 and the pipe 1. This setup may be described as a "cut-off regulator" for short. The cut-off regulator as proposed in the present invention constitutes a "wall 3" dividing the cross section of the pipe 1 into parts such that the part, enclosing volume 4, in front of the cavity resonator 2 becomes a waveguide with a cut-off frequency higher than the resonant frequency of the cavity resonator sensor 2. To make the description clearer, said cut-off regulator is shown roughly enclosed by dashed shape 5 in the figures. In the basic design the wall 3 has a negligible cross section seen along the pipe length and would therefore offer minimal restriction to the flow along the pipe.

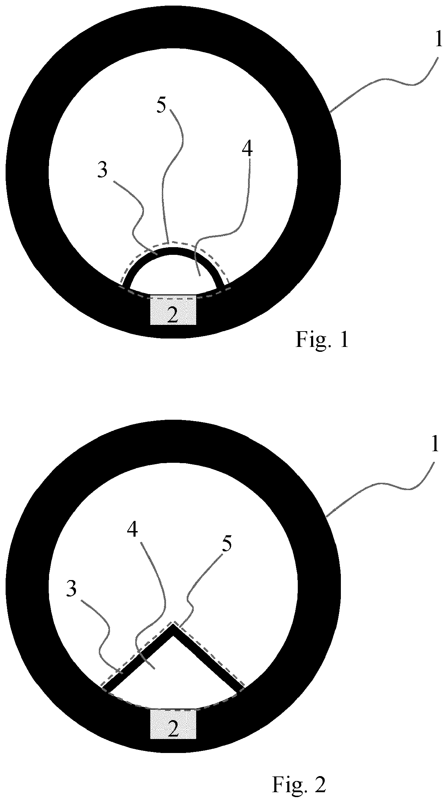

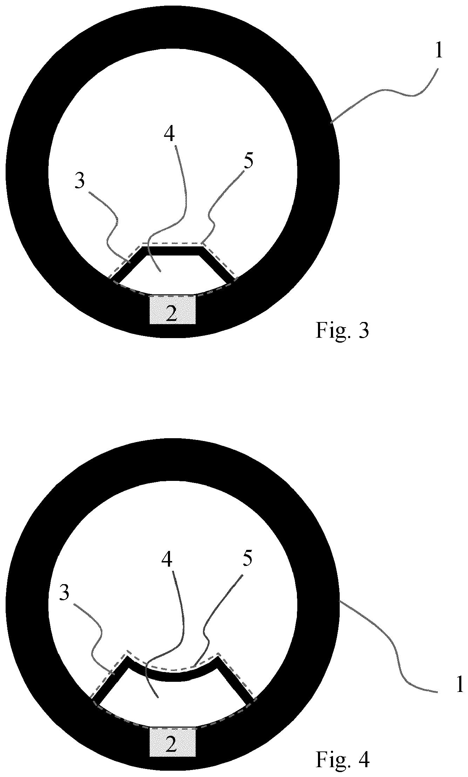

As is clear from figures 1-6, the shape of the cut-off regulator 5, or the shape enclosing volume 4, is not critical, as practically any shape will act as a waveguide, and can therefore be chosen based on other aspects, such as mechanical robustness, resistance to vibrations, ease in manufacturing, or by available standard materials. However, for shapes, which are not commonly used as waveguides, it may be very hard to find equations for calculating the cut-off frequency. In that case, computer based simulations of electromagnetic problems, e.g. HFSS or its likes can be used to find the cut-off frequency.

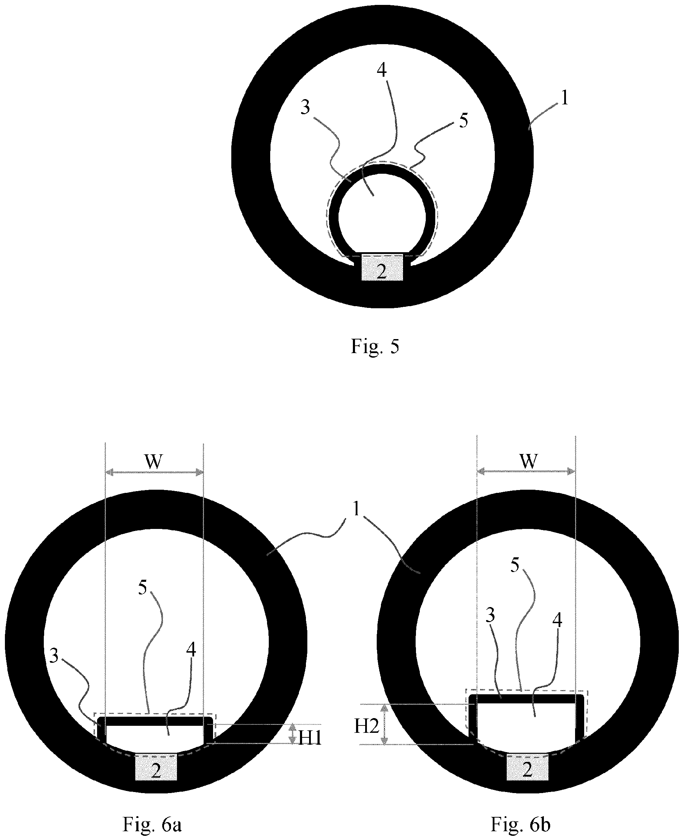

Figure 5 shows a typical embodiment of the present invention where the resonator 2 and plate 3 are introduced into the pipe 1 as an assembly. Such an assembly is introduced and installed into the pipe 1 from the inside of said pipe. Another feature of the embodiment shown in fig. 5 is that the waveguide is defined entirely by the plate 3 and the surface of the resonator 2 - the inner surface of the pipe 1 is not involved in defining the waveguide shape. The plate 3 shown in fig. 5 forms a circular cross-section of the regulator 5, however other geometrical shapes are also possible. Only a circular profile is shown for the sake of simplicity.

As a general rule, a small area of cross section of the cut-off regulator 5 means a high cut-off frequency, or more precisely, it is the largest dimension in the cross section, which defines the cut-off frequency rather than the area of cross-section. To illustrate it more clearly, we refer now specifically to figs. 6a and 6b. As an example, two cut-off regulators with rectangular shaped cross-sections will have nearly similar cut-off frequencies as long as the rectangular cross-sections have the same widths W, even though the heights H1 and H2 are different.

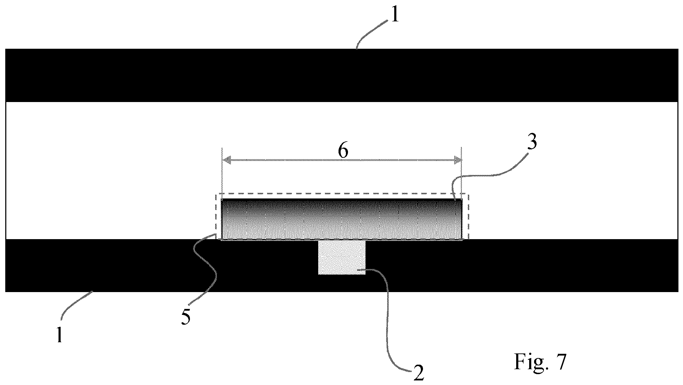

When electromagnetic fields are excited in a waveguide at a frequency below cut-off, as by the cavity resonator 2 in this case, the fields will not propagate, but be exponentially decaying with the distance from the point of excitation. If the waveguide is shorter than a certain length and has an open end, the fields will not be completely attenuated before reaching the open end. In the case of the cut-off regulator that would result in some loss of power by radiation into the pipe 1 from the open end of the waveguide or in this case, the cut-off regulator 5. As is illustrated in figure 7 the cut-off regulator therefore also needs to be of some minimum length 6 running along the length of the pipe 1 to prevent radiation from escaping the waveguide, or dedicated channel. The closer the resonant frequency is to the cut-off frequency the longer the cut-off regulator needs to be. In a typical case, a length (6) of 2 to 4 times the largest dimension in the aperture of the cavity resonator 2 is enough. The resonator 2 preferably sits roughly in the middle of the length 6 of the proposed waveguide.

To summarize, the invention relates to a sensor assembly for measuring dielectric properties in a hydrocarbon fluid flow in a pipe 1, especially measuring salinity of said flow. Said flow is traversing along the length of said pipe. The assembly comprising a microwave cavity resonator 2 being adapted to be positioned in the wall of said pipe 1 being designed to operate at a chosen resonant frequency so as to measure specific parameters such as the permittivity or salinity of the fluid flow present in the pipe.

The sensor assembly also comprising a metallic plate 3 at a distance from said sensor, wherein the plate defines a waveguide enclosing the cavity resonator sensor. By enclosing it is meant that said metallic plate 3 creates a dedicated channel within said pipe 1, along the length or axis of the pipe 1 with said sensor 2 located at chosen location on the periphery of said channel. Said channel acts as a waveguide for the microwave signals generated by said sensor 2. The waveguide is open along the axis or length of the pipe 1 for allowing said flow to pass through while having sufficient length 6 to avoid radiation loss through the openings at the ends. The waveguide defines a volume 4 between the cavity resonator 2, the plate 3 and the pipe wall 1, said waveguide having a cutoff frequency being higher than the resonant frequency of the cavity resonator sensor, the pipe wall in said volume also preferably being made from a metal. Preferably, the metal plate 3 extends along the length of said pipe at least two times the largest dimension along the aperture of the cavity resonator 2.

The waveguide may be realized in any suitable geometrical cross-section, such as, circular, elliptical, polygonal shapes, and their combinations
The cavity resonator 2 is adapted to have a surface or aperture facing said flow and being flush with the pipe wall while the metallic plate 3 extending into said flow but has a minimum area in the flow direction so as not to have any substantial effect on the flow conditions.

The metallic plate 3 can be mounted on said cavity resonator 2, for being introduced into said pipe 1 as a single unit or may be mounted in the pipe wall 1 at the position of the cavity resonator 2.

The sensor assembly may be a part of a sensor system further comprising a measuring means coupled to said sensor assembly for detecting the resonant frequency of the cavity resonator. Said measuring means may also measure the Q-factor of the resonator. The measuring means may further measure other electrical parameters related to the system. Said other electrical parameters may include, voltage and current amplitudes of the excitation to the cavity resonator, impedances, and phase of the electrical signals related to the resonator. The system may further comprise other measurements to improve the robustness of the system. Said other measurements may include the temperature measurements.

## Claims

1. Sensor assembly for measuring dielectric properties in a hydrocarbon fluid flow in a pipe (1), especially measuring salinity of said flow, comprising a microwave cavity resonator (2) being adapted to be positioned in the wall of said pipe (1), and being designed to operate at a chosen resonant frequency,
the sensor assembly also comprising a metallic plate (3) at a distance from said microwave cavity resonator (2), wherein the plate (3) defines a waveguide enclosing the microwave cavity resonator (2), the waveguide being open along the length of the pipe (1) for allowing said flow to pass through, the waveguide having a cutoff frequency being higher than the resonant frequency of the microwave cavity resonator (2).

2. Sensor assembly according to claim 1, wherein said metallic plate (3) extends along the length of said pipe (1) at least two times the largest dimension of the surface facing said flow of the microwave cavity resonator (2).

3. Sensor assembly according to claim 1, wherein said metallic plate (3) extends along the length of said pipe (1) at least four times the largest dimension of the surface facing said flow of the microwave cavity resonator (2).

4. Sensor assembly according to claim 1, wherein said waveguide is realized in geometrical cross-sections including, circular, elliptical, polygonal shapes, and their combinations.

5. Sensor assembly according to claim 1, wherein said microwave cavity resonator (2) is adapted to have a surface facing said flow and being flush with the pipe (1) wall, the metallic plate (3) extending into said flow.

6. Sensor assembly according to claim 1, wherein said metallic plate (3) is mounted on said microwave cavity resonator (2), for being introduced into said pipe (1) as a single unit.

7. Sensor assembly according to any of the above claims, wherein said waveguide excludes the wall of said pipe (1).

8. Sensor system including a sensor assembly according to any of the above claims, comprising measuring means coupled to said sensor assembly for detecting the resonant frequency of said cavity resonator (2).

9. Sensor system including a sensor assembly according to any of the above claims, wherein said measuring means also measures other parameters including, the Q-factor of said cavity resonator (2).

## Patentansprüche

1. Sensoranordnung zum Messen der dielektrischen Eigenschaften in einem Kohlenwasserstofffluidstrom in einem Rohr (1), insbesondere zum Messen des Salzgehalts des Stroms, einen Mikrowellenhohlraumresonator (2) aufweisend, der zur Anordnung in der Wand des Rohres (1) ausgelegt ist und der zum Betrieb bei einer gewählten Resonanzfrequenz konstruiert ist, wobei die Sensoranordnung auch eine Metallplatte (3) in einem Abstand von dem Mikrowellenhohlraumresonator (2) aufweist, wobei die Platte (3) einen Wellenleiter definiert, der den Mikrowellenhohlraumresonator (2) umschließt, wobei der Wellenleiter entlang der Länge des Rohres (1) offen ist, um den Durchfluss zu ermöglichen, wobei der Wellenleiter eine Ausschlussfrequenz aufweist, die höher ist als die Resonanzfrequenz des Mikrowellenhohlraumresonators (2).

2. Sensoranordnung nach Anspruch 1, wobei sich die Metallplatte (3) entlang der Länge des Rohres (1) um mindestens das Zweifache der größten Abmessung der Oberfläche erstreckt, die dem Strom des Mikrowellenhohlraumresonators (2) zugewandt ist.

3. Sensoranordnung nach Anspruch 1, wobei sich die Metallplatte (3) entlang der Länge des Rohres (1) um mindestens das Vierfache der größten Abmessung der Oberfläche erstreckt, die dem Strom des Mikrowellenhohlraumresonators (2) zugewandt ist.

4. Sensoranordnung nach Anspruch 1, wobei der Wellenleiter in geometrischen Querschnitten, darunter kreisförmige, elliptische, polygonale Formen und Kombinationen davon, realisiert ist.

5. Sensoranordnung nach Anspruch 1, wobei der Mikrowellenhohlraumresonator (2) dazu ausgelegt ist, eine dem Strom zugewandte Oberfläche aufzuweisen, die mit der Rohr-(1)-Wand bündig ist, wobei sich die Metallplatte (3) in den Strom hinein erstreckt.

6. Sensoranordnung nach Anspruch 1, wobei die Metallplatte (3) auf dem Mikrowellenhohlraumresonator (2) montiert oder als eine Einzeleinheit in das Rohr (1) eingeführt ist.

7. Sensoranordnung nach einem der obigen Ansprüche, wobei der Wellenleiter die Wand des Rohres (1) ausschließt.

8. Sensorsystem, eine Sensoranordnung nach einem der vorstehenden Ansprüche einschließend, Messmittel aufweisend, die mit der Sensoranordnung gekoppelt sind, um die Resonanzfrequenz des Hohlraumresonators (2) zu detektieren.

9. Sensorsystem, eine Sensoranordnung nach einem der obigen Ansprüche einschließend, wobei die Messmittel auch andere Parameter messen, darunter den Q-Faktor des Hohlraumresonators (2).

## Revendications

1. Ensemble capteur pour mesurer des propriétés diélectriques dans un écoulement de fluide hydrocarboné dans une conduite (1), en particulier pour mesurer la salinité dudit écoulement, comprenant un résonateur à cavité hyperfréquence (2) pouvant être positionné dans la paroi de ladite conduite (1), et étant conçu pour fonctionner à une fréquence de résonance choisie,
l'ensemble capteur comprenant également une plaque métallique (3) à distance dudit résonateur à cavité hyperfréquence (2), dans lequel la plaque (3) définit un guide d'ondes enfermant le résonateur à cavité hyperfréquence (2), le guide d'ondes étant ouvert sur la longueur de la conduite (1) pour autoriser ledit écoulement à passer de manière traversante, le guide d'ondes ayant une fréquence de coupure supérieure à la fréquence de résonance du résonateur à cavité hyperfréquence (2).

2. Ensemble capteur selon la revendication 1, dans lequel ladite plaque métallique (3) s'étend sur la longueur dudit tuyau (1) au moins deux fois la plus grande dimension de la surface en regard dudit écoulement du résonateur à cavité hyperfréquence (2).

3. Ensemble capteur selon la revendication 1, dans lequel ladite plaque métallique (3) s'étend sur la longueur dudit tuyau (1) au moins quatre fois la plus grande dimension de la surface en regard dudit écoulement du résonateur à cavité hyperfréquence (2).

4. Ensemble capteur selon la revendication 1, dans lequel ledit guide d'ondes est réalisé en sections géométriques comprenant des formes circulaire, elliptique, polygonale et des combinaisons de celles-ci.

5. Ensemble capteur selon la revendication 1, dans lequel ledit résonateur à cavité hyperfréquence (2) est adapté pour avoir une surface en regard dudit écoulement et affleurant la paroi de la canalisation (1), la plaque métallique (3) s'étendant dans ledit écoulement.

6. Ensemble capteur selon la revendication 1, dans lequel ladite plaque métallique (3) est montée sur ledit résonateur à cavité hyperfréquence (2), pour être introduite dans ladite canalisation (1) sous la forme d'une unité unique.

7. Ensemble capteur selon l'une quelconque des revendications précédentes, dans lequel ledit guide d'ondes exclut la paroi de ladite canalisation (1).

8. Système de capteur comprenant un ensemble capteur selon l'une quelconque des revendications précédentes, comprenant des moyens de mesure couplés audit ensemble de capteur pour détecter la fréquence de résonance dudit résonateur à cavité hyperfréquence (2).

9. Système de capteur comprenant un ensemble capteur selon l'une quelconque des revendications précédentes, dans lequel lesdits moyen de mesure mesurent également d'autres paramètres, y compris le facteur Q dudit résonateur à cavité hyperfréquence (2).
